# EUROPEAN PATENT APPLICATION

(11) **EP 1 947 196 A1**
(43) Date of publication of application: **23.07.2008**
(21) Application number: 06823208.1
(22) Date of filing: 08.11.2006
(51) Int. Cl.: C12Q 1/68, C12N 15/09

(54) **METHOD OF AMPLIFYING MULTIPLE NUCLEIC ACID SEQUENCES FOR DIFFERENTIATION**

(30) Priority: 08.11.2005 JP 2005323726
(71) Applicant: Olympus Corporation, Tokyo 151-0072 (JP)
(72) Inventor: TANABE, Tetsuya c/o Intellectual Property Support Dept., Hachioji-shi, Tokyo 192-8512 (JP); MORIMOTO, Nobuhiko c/o Intellectual Property Support Dept., Hachioji-shi, Tokyo 192-8512 (JP)
(74) Representative: von Hellfeld, Axel
(86) International application number: PCT/JP2006/322304
(87) International publication number: WO 2007/055255

(57) **Abstract**

The invention provides a method with fewer distribution of the amount of amplification among plural tags when the plural tags are used for multiplex detection using the tag. A method for amplifying plural nucleic acid fragments, the nucleic acid fragment including one nucleic acid sequence for discrimination selected from a first group consisting of first to n-th nucleic acid sequences for discrimination and one nucleic acid sequence for amplification selected from a second group consisting of first to m-th nucleic acid sequences for amplification, the method including amplifying a mixture of nucleic acid fragments containing plural kinds of the nucleic acid fragments under a condition capable of amplifying the nucleic acids using, as primers, at least a part of the sequences of all the nucleic acid sequences for discrimination that may exist in the mixture of the nucleic acid fragments, and sequences complementary to at least a part of the sequences of the nucleic acid sequences for amplification or sequences at the same strand side as the nucleic acid sequences for amplification, wherein n is an integer of 2 or more, the first to n-th nucleic acid sequences for discrimination contained in the first group have different nucleotide sequences from one another, m is an integer from 1 or more to n or less, and the first to m-th nucleic acid sequences for amplification contained in the second group have different nucleotide sequences from one another.

## Description

### Technical Field

The present invention relates to an amplification reaction technique of nucleic acids.

### Background Art

SNP (single nucleotide polymorphism) is polymorphism of genes that is found in a frequency of one nucleotide in hundreds of nucleotides. Such mutation is widely distributed on a genome irrespective of coding regions and non-coding regions. Mutation of the gene includes substitution of a nucleotide as well as insertion and deletion of the nucleotides. Since the human genome has a size of 300 x 10⁶ base pairs, the number of SNP accounts for 3 x 10⁶ in total provided that the proportion of polymorphism is one nucleotide per 1000 nucleotides. Accordingly, it is not an easy task to find medically useful SNP among such vast number of SNPs. For example, related SNPs may be specified with higher accuracy by detecting as many samples as possible for elucidating the correlations between SNP and diseases or SNP and drug sensitivity. For this purpose, case groups and control groups in numbers enough for obtaining statistically significant results are constructed. Then, SNPs suspicious of correlations are detected with a density and number necessary for each group, and the types of these SNPs are determined, namely, subjected to typing. Furthermore, analysis of correlations to diseases or drug sensitivity is necessary thereafter.

Sanger's method is used for typing using a DNA sequencer. While neighboring nucleotide sequences may be decided with identification of insertion and deletion by sequencing, it is quite difficult to design primers for amplifying genes having SNPs, and the reagents for the sequencing reaction and the sequencing apparatus are expensive. The number of SNPs capable of typing by one time of sequencing is about one or two on average in terms of average distances of SNPs and nucleotide length available for sequencing. The number of SNPs capable of being tested by one run is restricted in the highly reliable Sanger's method that requires much time and cost. Accordingly, the target genes are forced to be confined at the time of typing in the studies for elucidating the relation between the disease and SNP.

The multiplex method has been proposed since the latter half of 1990's as a method for analyzing genes such as SNPs.

For example, Barany et al. of Cornell University have developed a method for detecting plural SNPs in the same reaction solution by combining a method for detecting SNP using ligase referred to as LDR (ligase detection reaction) and conversion of a desired nucleic acid sequence into a tag called a zip code. This method is currently commercially available as a detection kit SNPlex manufactured by ABI (Jpn. Pat. Appln. KOKAI Publication Nos. 2000-511060, 2001-519648 and 2004-526402).

Orchid Cellmark has provided a method referred to as SNP-IT for distinguishing 48 alleles in a microarray disposed at the bottom of a microplate by a detection reaction in one reaction solution (Japanese Patent No. 3175110 and Jpn. Pat. Appln. KOKAI Publication No. 2002-508664).

A method provided by Illmina is thought to be most successful today. In this method, the desired nucleic acid sequence is converted into a zip code sequence after detecting SNP by a polymerase elongation reaction and a ligation reaction of ligase. 1500 zip code sequences in maximum are simultaneously detected by using a unique detection device (referred to as "bead array") similar to the microarray system (Jpn. Pat. Appln. KOKAI Publication Nos. 2002-519637 and 2003-521252).

TM Bioscience has provided a multiplex reaction kit for detection taking advantage of beads colored with fluorescent reagents manufactured by Luminex (Jpn. Pat. Appln. KOKAI Publication Nos. 2004-522440 and 2004-526433). This kit is provided as a genetic disease detection kit in the laboratory.

Parellele has provided a MIP (Molecular Inversion Probe) method. In this method, the synthesis cost of the probe is reduced by taking advantage of a cyclic probe and a gap ligation method, and a multiplex typing method is provided by using a tag with enhanced reaction efficiency (Hardenbol, P. et al., Nat. Biotechnol. 21, 673-678 (2003); Hardenbol, P. et al., Genome Res. 15, 269-675 (2005)).

The multiplex technology is usually configured to dispose common primers so that the common primers are adjoining to both sides of the tag, or so that the tag is interposed between other sequences. The multiplex method (also referred to as a tag multiplex method) taking advantage of the tags has been proposed as effective means for analyzing SNP.

The multiplex method using the tag includes a reaction for converting a natural gene sequence into an artificial sequence called a tag, and a process for detecting the tag after conversion. In the multiplex reaction, the tag sequence has two features for converting plural genes into a tag in one-to-one correspondence in a reaction solution and for detecting the tag. The first feature is that respective tags independently react without cross-hybridization to one another. The second feature is that the tags are designed so that the melting points (or Tm values) are approximately equal to one another in order to permits the tags to simultaneously react in the same solution. The tags may be freely correlated to genes by the multiplex method, different from the microarray that traps gene sequences themselves with probes. Accordingly, the same tag may be always detected in the detection step, and the method is flexible since the same detection means and detection device may be used even when the gene as a detection object is changed.

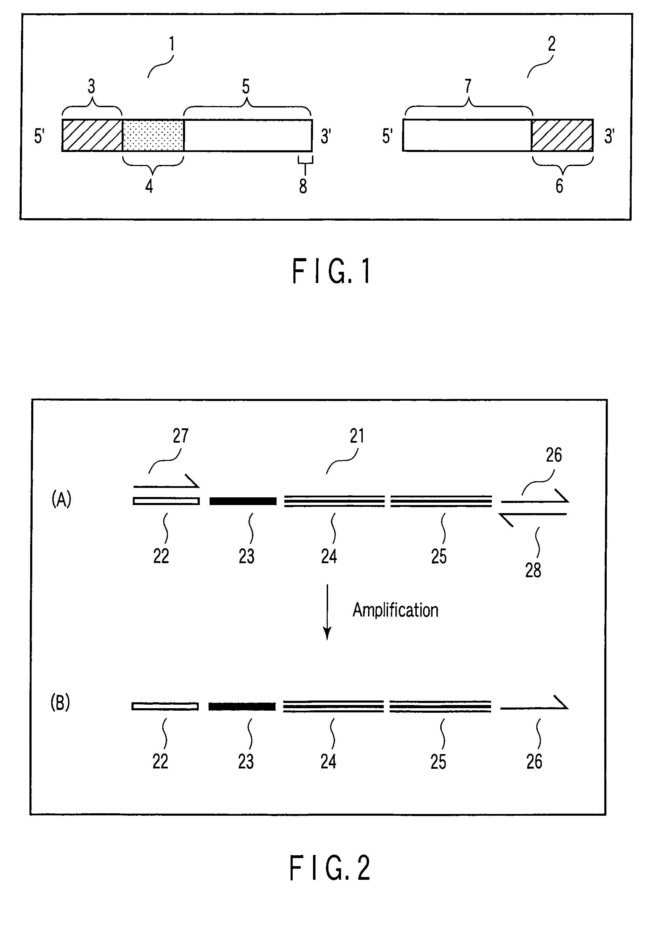

The tag multiplex method in the related art as described above includes amplifying the tag by PCR amplification using common primers contained together with the tag. The tag is detected after the amplification. An example of a nucleic acid for detection used in the related art is shown in FIG. 1. Two nucleic acids for detection are used in this case. The nucleic acid 1 for detection has, from the 5'-side, a common primer sequence 3, a nucleic acid tag 4 for discrimination and a complementary sequence 5 of a test gene. The nucleic acid 2 for detection has, from the 3'-side, a common primer sequence 6 and a complementary sequence 7 of a test gene. For example, when the nucleic acids 1 and 2 for detection are used for the zip code method, a nucleotide 8 complementary to SNP to be detected is further contained at the 3'-end of the detection nucleic acid 1; primer sequences contained in the nucleic acid 1 for detection and nucleic acid 2 for detection, respectively, which have been designed to be adjoining to one another are used; and the tag is amplified in a manner dependent on the presence of the nucleic acid to be detected. The nucleic acid for detection is used not only in the zip code method but also in many tag multiplex methods in the related art, and the tag is amplified depending on the presence of the nucleic acid to be detected.

The amplification rate becomes uneven and the amount of the amplification product obtained is not uniform in the multiplex method using the tag sequence in the related art, such that the amplified tag sequence is biased to a specific tag sequence with a high amplification rate when the tag sequence is amplified by PCR or, conversely, the amplification rate is low with respect to a specified tag sequence.

### Disclosure of Invention

### [Problems to be Solved by the Invention]

In view of the foregoing circumstances, an object of the invention is to provide a method for amplifying plural kinds of nucleic acids for discrimination.

Another object of the invention is to provide a method for amplifying plural tags with fewer distribution in the amount of amplification in the multiplex detection using the tag.

### [Means for Solving the Problems]

In order to achieve the above object, the present invention provides the following means. That is:
(1) A method for amplifying plural nucleic acid fragments,
   the nucleic acid fragment comprising one nucleic acid sequence for discrimination selected from a first group consisting of first to n-th nucleic acid sequences for discrimination and one nucleic acid sequence for amplification selected from a second group consisting of first to m-th nucleic acid sequences for amplification,
   the method comprising amplifying a mixture of nucleic acid fragments containing plural kinds of the nucleic acid fragments under a condition capable of amplifying the nucleic acids using, as primers, at least a part of the sequences of all the nucleic acid sequences for discrimination that may exist in the mixture of the nucleic acid fragments, and sequences complementary to at least a part of the sequences of the nucleic acid sequences for amplification or sequences at the same strand side as the nucleic acid sequences for amplification,
   wherein n is an integer of 2 or more, the first to n-th nucleic acid sequences for discrimination contained in the first group have different nucleotide sequences from one another, m is an integer from 1 or more to n or less, and the first to m-th nucleic acid sequences for amplification contained in the second group have different nucleotide sequences from one another.
(2) A reagent kit used for the method according to (1), containing plural primers from the first to n-th primers, common primers, an enzyme for amplifying the nucleic acids, a buffer component and a dNTP mixture.

### Brief Description of Drawings

FIG. 1 schematically illustrates a detection nucleic acid used in the method in the related art.
FIG. 2 schematically illustrates an amplification method in the related art using two common probes.
FIG. 3 schematically illustrates the concept of the invention.
FIG. 4 illustrates a scheme showing an example of a gene analysis method taking advantage of the method of the invention.
FIG. 5 illustrates a scheme showing an example of the gene analysis method of the related art.
FIG. 6 schematically illustrates a detection nucleic acid of the related art.
FIG. 7 schematically illustrates a detection nucleic acid of the related art.
FIG. 8 schematically illustrates a detection nucleic acid of the related art.
FIG. 9 schematically illustrates a detection nucleic acid of the related art.
FIG. 10 schematically illustrates the reaction of the detection nucleic acid in FIG. 8.
FIG. 11 is a graph showing the result of detection performed in examples.
FIG. 12 shows a scattergram by common primer PCR and a scattergram by tag mix PCR.
FIG. 13 shows a scattergram by common primer PCR and a scattergram by tag mix PCR.

### Best Mode for Carrying Out the Invention

### 1. Method for amplifying nucleic acid for discrimination

The concept of the method for amplifying plural kinds of nucleic acids for discrimination will be briefly described. FIG. 2 schematically illustrates a commonly used method in the related art. While the method will be described by focusing on one nucleic acid fragment for the convenience of illustration, plural nucleic acid fragments are subjected to the reaction when the method is practically applied. See FIG. 2A. Two common primers are used in the method in the related art as described above. Accordingly, a nucleic acid fragment 21 includes a nucleic acid sequence 23 for discrimination, a first common primer junction 22, a complementary sequence 24 of a first gene, a complementary sequence 25 of a second gene, and a second common primer junction 26. A first common primer 27 and a second common primer 28 are used for amplification of the nucleic acid fragment 21, which is amplified under a condition capable of amplification of the nucleic acid (FIG. 2A). A part of the amplification product thus obtained is a nucleic acid fragment as shown in FIG. 2B.

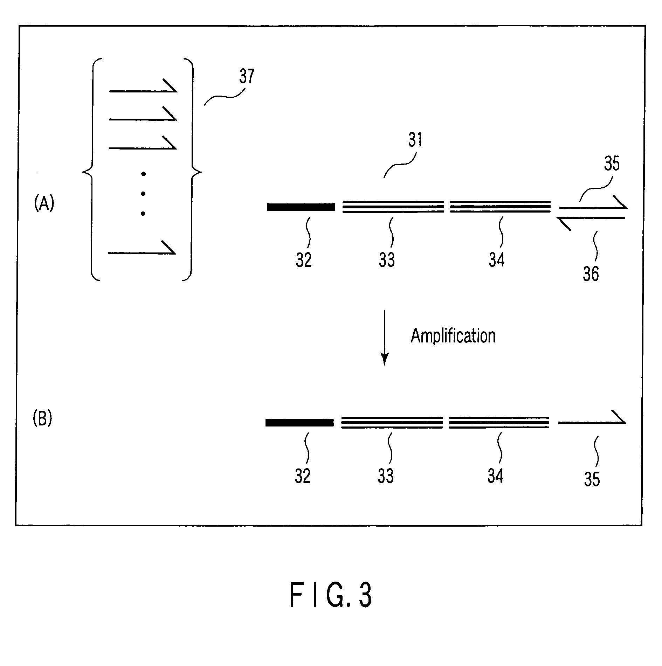

The concept of the method for amplifying plural kinds of nucleic acids for discrimination according to the invention will be briefly described. While the method will be described by focusing on one nucleic acid fragment for the convenience of illustration, plural nucleic acid fragments are subjected to the reaction when the method is practically applied. See FIG. 3A. A nucleic acid fragment 31 includes a nucleic acid sequence 32 for discrimination, a complementary sequence 33 of a first gene, a complementary sequence 34 of a second gene, and a nucleic acid sequence 35 for amplification. A common primer 36 having a complementary sequence to the nucleic acid sequence 35 for amplification and a mixture 37 of all the nucleic acid primers for discrimination are used for amplification of the nucleic acid fragment 31, which is amplified under a condition capable of amplifying the nucleic acid (FIG. 3B).

While the nucleic acid fragment of the related art contains the first common primer junction and second common primer junction for hybridizing by using two kinds of common primers (see FIG. 2A), the nucleic acid fragment used in the invention contains no sequence corresponding to the first common primer junction 22 shown in FIG. 2A (see FIG. 3A). As shown in FIG. 3A, the nucleic acid fragment according to an aspect of the invention contains a nucleic acid sequence 32 for discrimination, nucleic acid primers 37 for discrimination having a complementary or a partially complementary sequence to the nucleic acid sequence 32 for discrimination are used, and the primers are hybridized to the nucleic acid sequence 32 for discrimination depending on the sequences. While additional primers are necessary for amplification, the primer may be the common primer 36 having a sequence capable of hybridizing to the nucleic acid sequence 35 for amplification. For example, the sequence may be a sequence complementary to or partially complementary to the nucleic acid sequence for amplification, or may be a sequence with at least a part thereof homologous.
The "nucleic acid sequence for amplification" used in an aspect of the invention corresponds to the "common primer junction" in the related art, and the primer hybridized to the sequence may be referred to as a "common primer". Since the primer serves as a primer for hybridizing with the "nucleic acid for amplification", the primer may be referred to as a "nucleic acid primer for amplification", and the terms "common primer" and "nucleic acid primer for amplification" may be used interchangeably to one another.

The "common primer" as used in the invention has a nucleotide sequence selected from the nucleotide sequences of the nucleic acid sequence for amplification, and may contain each amplification sequence from a specified nucleotide sequence throughout all the nucleic acid fragments or a mixture of the plural nucleic acid fragments that commonly contain the specified nucleotide sequence. This permits the common primer to be commonly used for plural kinds of the nucleic acid fragment. The common primer may also have a sequence that completely or partially hybridizes with one nucleic acid sequence for amplification in an aspect of the invention, and plural kinds of the nucleic acid fragment may be simultaneously used in an amplification system of one nucleic acid for discrimination by adding different labels. For example, the common primer may be designed so as to discriminate the difference in genotypes at the polymorphic site to which the common primer is hybridized by labeling the primer so that it is able to be discriminated.

While only one of the "common primer junctions" as a "nucleic acid sequence for amplification" in an aspect of the invention is contained in the nucleic acid fragment, one or more of the common primers used for hybridization may be simultaneously used. Preferably, the number of the common primers is one or more and less than the number of the kinds of the nucleic acid primers for discrimination contained in the mixture of all the nucleic acid primers for discrimination used together.

While FIG. 3 shows an example using one nucleic acid fragment for the convenience of illustration, a mixture of all the nucleic acid primers for discrimination having complementary sequences to at least a part of the nucleic acid for discrimination is used as the primer, and plural nucleic acid fragments may be subjected to an amplification reaction using at least one common primer.

All the amount of respective primers contained in the mixture of all the nucleic acid primers for discrimination may be equal, and the proportion of the corresponding nucleic acid primer for discrimination contained in the mixture of all the nucleic acid primers for discrimination may be changed depending on the amplification rate of the nucleic acid for discrimination in concern.

Dispersion of the amplification rate for each nucleic acid amplified is remarkably reduced as compared with the rate in the related art by the method for amplifying plural kinds of nucleic acid for discrimination according to the invention.

Since each nucleic acid for discrimination is amplified with a primer complementary to each nucleic acid for discrimination in an aspect of the invention, the amount of the primer for amplifying each nucleic acid for discrimination is restricted and amplification of the tag having a high amplification efficiency is prevented from being abnormally enhanced. On the other hand, a nucleic acid for discrimination having a low amplification rate is not deprived of its own primer to be used for amplification by the nucleic acid for discrimination having a high amplification rate, and an amplification product of the former nucleic acid is formed. Accordingly, plural kinds of the nucleic acid for discrimination may be amplified with good balance.

Since the common primer is not necessary at one site where the common primer has been necessary in the related art, the length of the nucleic acid fragment is reduced. Consequently, the cost for synthesizing the nucleic acid may be reduced while the time necessary for synthesis and/or purification may be shortened.

While the "nucleic acid fragment" as used herein may be an oligonucleotide conveniently synthesized according to the request of an operator, may be composed of a natural nucleic acid, or may partly contain the natural nucleic acid, the fragment is preferably an oligonucleotide synthesized according to the request of the operator. The "nucleic acid fragment" may be referred to as a "nucleic acid for detection" in the tag multiplex method according to an aspect of the invention to be described below, and the two terms "nucleic acid fragment" and "nucleic acid for detection" may be used interchangeably. The length of the nucleic acid fragment is from about 10 nucleotides to about 50 nucleotides, preferably from about 15 nucleotides to about 30 nucleotides.

The "mixture of the nucleic acid fragment" as used herein refers to a mixture containing plural nucleic acid fragments. In particular, the nucleotide sequences of the nucleic acid fragment contained in the "mixture of the nucleic acid fragment containing plural kinds of the nucleic acid fragments" are composed of two or more sequences.

The term used in the "nucleic acid for discrimination" refers to a nucleic acid as an object of amplification contained in the nucleic acid fragment. For example, the term includes a nucleic acid that contains a sequence as an object of detection in the process following the amplification or a sequence as an object of detection in the process of amplification. When the method is used in the tag multiplex method as an aspect of the invention to be described below, the terms "nucleic acid for discrimination" and "tag" may be used interchangeably. The terms "tag" and "nucleic acid tag for discrimination" may be also used interchangeably. The length of the nucleic acid for discrimination is from about 12 nucleotides to about 50 nucleotides, preferably from about 15 nucleotides to about 30 nucleotides.

The term "a mixture of all the nucleic acid primers for discrimination" may be used interchangeably with the term "tag mix primer" when the term "nucleic acid for discrimination" refers to the "tag". The nucleic acid used as a primer by hybridizing with the "nucleic acid for discrimination" is referred to as a "nucleic acid primer for discrimination" or simply a "primer for discrimination".

While the number of the primers contained in a mixture of the nucleic acid primer for discrimination used in a reaction system may be determined depending on the request of the operator of the method according to the invention, the kind and amount of the primer contained in the reaction system preferably correspond to the number of sequences for discrimination and amplification that has been determined in advance.

The number of the nucleotides of the nucleic acid sequence for discrimination may be the same as the number of the nucleotides of the corresponding primer, or the former may be 1 to 10 nucleotides longer than the latter.

The number of the nucleotides of the nucleic acid sequence for amplification may be the same as the number of the nucleotides of the corresponding common primer, or the former may be 1 to 10 nucleotides longer than the latter. The length of the nucleic acid sequence for amplification is from about 10 nucleotides to about 50 nucleotides, preferably from about 20 nucleotides to about 30 nucleotides.

The term "nucleic acid" as used herein refers to all the DNAs and RNAs including cDNA, genome DNA, synthetic DNA, mRNA, whole RNA, hnRNA and synthetic RNA.

While the "condition capable of amplification of the nucleic acid" as used herein may be any conditions capable of amplifying the nucleic acid, per se, an appropriate condition, a more appropriate condition or a most appropriate condition for executing the amplification reaction may be freely selected by the operator of the invention. For example, the condition capable of amplifying the nucleic acid may include appropriate primers according to the invention, any enzymes for amplifying the nucleic acid known in the art, per se, any buffer components known in the art, per se, for adjusting a salt concentration balance in the reaction solution and a dNTP mixture, and a reaction temperature suitable for amplification may be maintained under the condition.

While the terms "amplification" as used herein may be any amplification methods known in the art, per se, amplification methods such as PCR and asymmetric PCR may be preferably used.

### 2. Multiplex tag amplification method

According to another aspect of the invention, the amplification method of the invention may be used for advantageously amplifying a tag in any of nucleic acid reaction systems performed by taking advantage of known tags, per se, in a multiplex detection method and in an amplification method of the sequence in DNA computing using the tag.

For example, a multiplex typing method taking advantage of the multiplex detection method has been proposed sine the latter half of 1990s in order to comply with the requirements of low cost and high throughput of gene typing.

According to an aspect of the invention, distribution of the amount of amplification that has been a problem in amplification of the tag may be conveniently suppressed by using the method in the above-mentioned paragraph "1. method for amplifying nucleic acid for discrimination".

The inventors of the invention have noticed that not only the tag but also gene sequences containing SNP are amplified together in a region between the primer sequences when the tag contained in the nucleic acid for detection is amplified in the multiplex detection method. As a result, it was also found that the amplification rate becomes irregular due to amplification of sequences other than the tag, which causes distribution of the amount of amplification. In addition, even when the reaction method is devised so that only the tag sequence is amplified by selecting the sequence so that the amplification efficiency is uniform, the amount of amplification may be different at the initial stage of amplification of the tag due to the difference in efficiency of the tag exchange reaction before the amplification efficiency becomes uniform. The inventors of the invention have found a multiplex tag amplification method according to the invention based on the discoveries as described above.

The "multiplex tag amplification method" as used herein refers to a method for amplifying the tag by using primers having a complementary sequence to each of plural tags as a mixed primer.

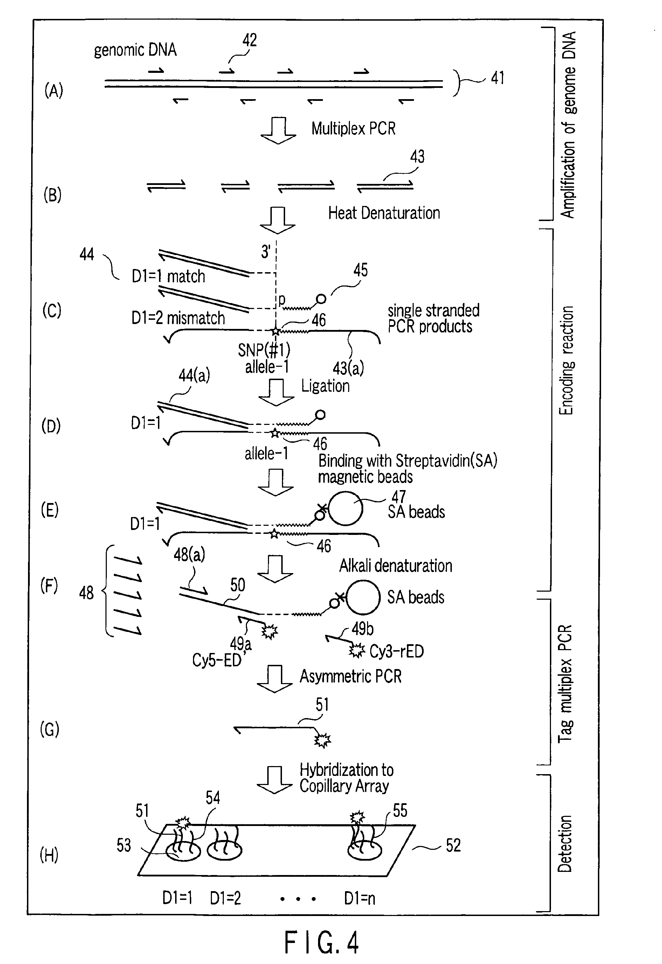

An example of the SNP analysis method taking advantage of the multiplex tag amplification method according to an aspect of the invention will be described below with reference to FIG. 4.

Mutation sites to be analyzed, for example genome DNAs that contain SNP, are sampled from a subject, and are subjected to multiplex PCR (referred to as "Multiplex PCR" in the drawing) using plural primers 42 (FIG. 4A). PCR products 43 obtained are thermally denatured (referred to as Heat Denaturation in the drawing: FIG. 4B). A single strand PCR product 43a obtained contains SNP (#1) 46 in one of the chains.
The single strand PCR product 43a, an oligonucleotide group 44 containing a tag sequence that has been designed in advance, and a primer 45 labeled with biotin are allowed to react under an appropriate condition (FIG. 4C). When the genotype of SNP 46 under analysis matches the sequence of an oligonucleotide 44a contained in the oligonucleotide group 44, they are linked by ligation (referred to as "Ligation" in the drawing: FIG. 4D), and the ligation product is bonded to magnetic beads 47 having immobilized streptavidin (referred to as "SA" in the drawing: FIG. 4E). The ligation product is denatured with an alkali (referred to as "Alkali Denaturation" in the drawing), and a linked nucleotide 50 is amplified under a condition capable of asymmetric PCR amplification (FIG. 4F) in the presence of a tag mix primer 48, a first fluorescence-labeled common primer 49a that is a common primer labeled with a first fluorescent substance and a second fluorescence-labeled common primer 49b that is a common primer labeled with a second fluorescent substance while the ligation product is bonded to the magnetic beads 47. Since the first fluorescent substance can be discriminated from the second fluorescent substance, the genotype of target SNP may be discriminated by using these fluorescent substances. Consequently, a PCR product 51 labeled with either the first or second fluorescent substance depending on the genotype may be obtained by using an appropriate tag primer 48a (FIG. 4G). When the fluorescence-labeled PCR product 51 is allowed to react with a microarray 52 having a probe 54 complementary to each tag in a prove immobilized region 53, the PCR product hybridizes with the probe in the probe-immobilized region 53 at D1 = 1 (FIG. 4H). The allele of SNP (#1) at a specified site may be determined by measuring the fluorescence intensity of the fluorescent label.

The desired product may be specifically amplified with uniform amplification efficiency by the method according to an aspect of the invention even for a sample that contains many complex sequences and similar sequences such as human genomes.

The "nucleic acid" refers to all the DNAs and RNAs including cDNA, genome DNA, synthetic DNA, mRNA, whole RNA, hnRNA and synthetic RNA in the specification of the invention. While the target nucleic acids to be detected or quantified may be arbitrary nucleic acids having arbitrary sequences, nucleic acids that may serve as disease markers such as causative genes of genetic diseases, cancer-related genes or virus-derived nucleic acids are particularly preferable target nucleic acids. Accordingly, while the sample includes a body fluid such as blood, urine and saliva, arbitrary samples other than the body fluid may be also used.
The sample may be dissolved in a liquid by an appropriate method such as enzyme treatment or addition of a surfactant or an organic solvent when the sample is a solid. The target nucleic acid may be freely changed for implementing the method of the invention in addition to the above-mentioned method. For example, the detection reaction may be directly initiated from the genome DNA by large scale cultivation of an established cell strain or by preparing the human genome DNA necessary for the method of the invention in a large scale by acquiring a larger quantity of the peripheral blood. Alternatively, the detection reaction may be initiated from a sample in which the genome DNA is nonspecifically amplified by a WGA (Whole Genome Amplification) method using, for example, a reagent kit GenomiPhi (trade name; manufactured by Amersham Bioscience) after acquiring a small amount genome DNA. The detection reaction may be initiated from a sample in which a specific sequence is amplified by a method using a primer such as PCR, multiplex PCR or asymmetric PCR method. While the target nucleic acid is supposed to be a genome DNA when the method of the invention is used for detecting a SNP-specific sequence, the region that contains target SNP may be amplified by PCR in this case. The sample obtained by enzymatic amplification may be detected after converting the DNA into a single strand DNA by quenching to 4°C after heating to 95°C, or after applying an operation for converting into a single strand DNA or for fragmenting operation by, for example, fragmentation by heating to 95°C in a solution having a quite low salt concentration, by ultrasonic fragmentation or by digestion with a restriction enzyme.

A generally used PCR reaction solution may be used for the reaction solution used in the amplification process, and a commercially available kit may also be used. For example, the reaction solution may contain 0.1 µM each of an enzyme Titanium Taq, Titanium Taq Buffer (manufactured by Takara Bio) and primer, and 10 ng of template DNA with a reaction volume of 20 µL.

The following is an example of the embodiment of the invention. The following is use and situation of the detection method of the invention. For example, the method of the invention may be used for research purposes such as elucidation of correlation between human genotype and diseases, detection of drug sensitivity, detection of the change of bonding of proteins in a gene control region, and molecular biological analysis of gene polymorphism by changing the object from human to a different organism. These researches may be implemented in the laboratories of academic institutes and laboratories of companies. When the correlation between a gene and a specified disease, morbidity risk and drug sensitivity are elucidated, the method of the invention may be used for clinical tests for selecting therapeutic methods at a clinical test center of a hospital, for preventive diagnosis by thorough medical examination, and for drug sensitivity test for selecting an anticancer agent with smaller side effects.

Other examples of the implementation of the method of the invention include: implementation as a research and diagnostic reagent kit for detecting gene polymorphism for implementing the method of the invention by the user himself; implementation by an automatic reactor for automatic treatment; and implementation for diagnosis at a research center on commission or clinical test center in place of implementation by the user and subject.

The conversion reaction to a tag is crucial in gene analysis. Examples of the reaction used for conversion include an oligonucleotide ligation assay (OLA) and elongation of one nucleotide using a polymerase. An ability of the enzyme to discriminate mismatch hybrid is utilized at best in these methods, and the sequence of the probe is determined so that the nucleotide of SNP is disposed at the 3'-terminal of the ligated probe in the ligase reaction (Luo, J. et al., Improving the fidelity of Thermus thermophilus DNA ligase, Nucl. Acids Res. 24, 3071-78, 1996).

For example, as many SNPs as possible need to be detected from patient groups and control groups, respectively, involving as many individuals as possible in order to elucidate the relation between the disease and SNP or between drug sensitivity and SNP. Thus, the relation between them may be found with high accuracy.

While two common primers are used in the related art, the same quantity of two kinds of primers of the sequence for amplification and a mixture of primers containing the same quantity of all kinds of sequences (or tags) for discrimination are subjected to PCR amplification as a set of primers in the reaction according to the embodiment of the reaction. However, the amount of the primer may be changed for each tag.

The amount of the primer for amplifying each tag is restricted in order to independently amplify each tag with a primer complementary to each tag. This prevents amplification of a tag having a high amplification efficiency from being abnormally enhanced. On the contrary, since a tag having a low amplification rate is not deprived of the primer to be used for its amplification by the tag having a high amplification rate, a sufficient amount of the amplification product is obtained. Accordingly, the amounts of amplification of plural tags may be suppressed from being distributed.

While gene analysis may be multiplexed using DCN in widely used SNP analysis using a DNA computer according to the invention, it is desirable in terms of the cost and work efficiency to multiplex PCR as well for cleaving the periphery of SNP from the genome to be subjected to pre-treatment. However, the invention is not restricted to this method.

The method according to the invention enables SNP to be effectively analyzed.

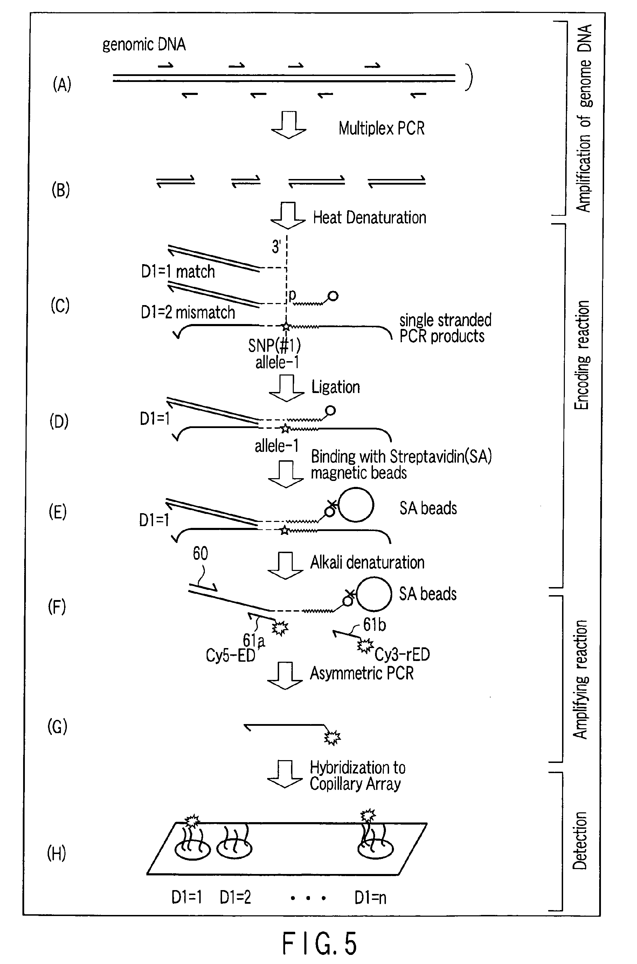

A scheme for performing an aspect of the invention shown in FIG. 4 by a method in the related art is illustrated in FIG. 5 for the purpose of reference.
The steps from FIGS. 5A to 5E are the same as those shown in FIG. 4. On the contrary, the amplification reaction in FIG. 5F differs from that in FIG. 4. While the primers having mutual sequences of the tag have been used in the method of the invention shown in FIG. 4, a common primer 60, a first labeling common primer 61a and a second labeling common primer 61b are used in FIG. 5.

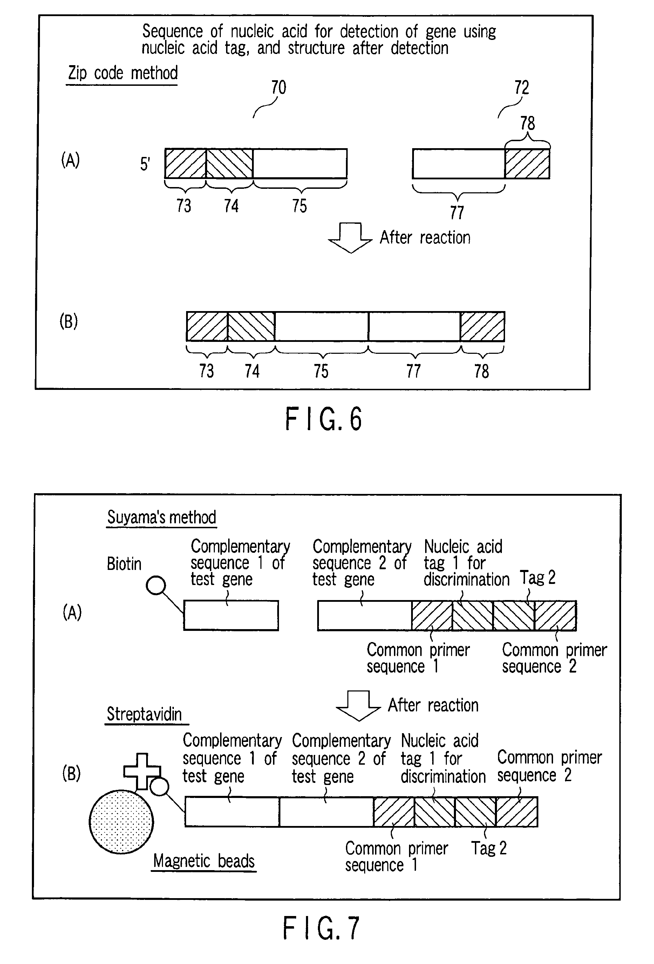

The amplification method of the nucleic acid for discrimination and/or tag multiplex method of the invention may be used for a zip code method shown in FIG. 6. The nucleic acid for detection used in the method is schematically illustrated. The desired gene is analyzed using a nucleic acid 70 for detection and a nucleic acid 72 for detection before reaction in the method shown in FIG. 6A. The nucleic acid 70 for detection is linked to the nucleic acid 72 for detection after the reaction (FIG. 6B).

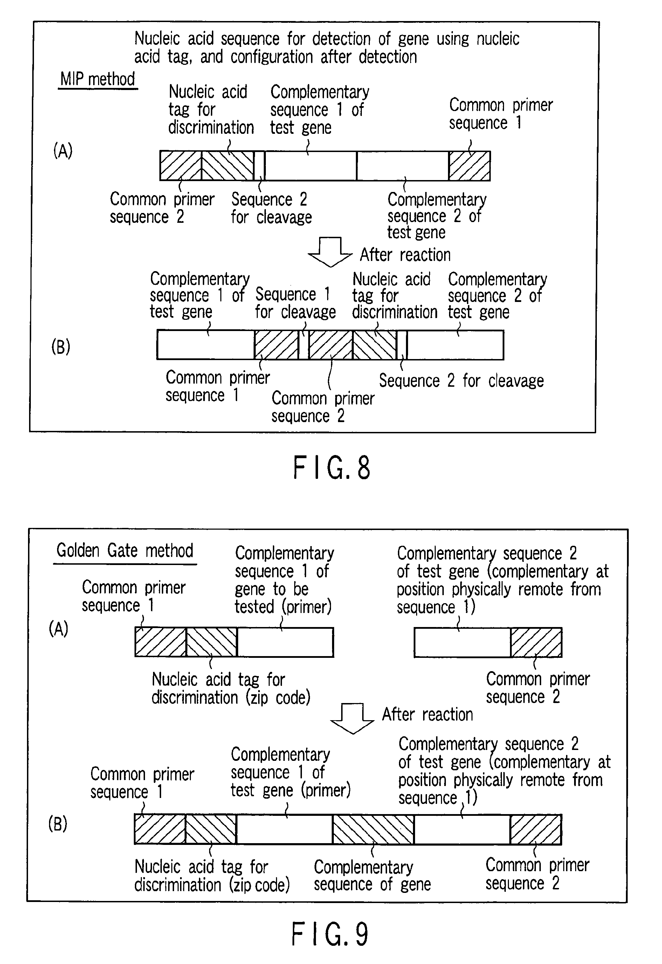

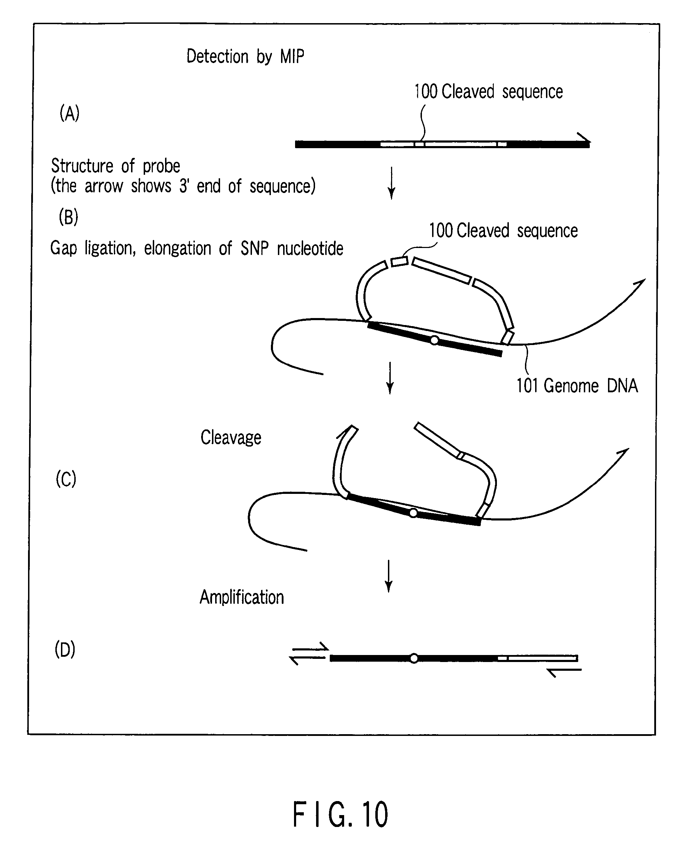

FIG. 7 shows the state (FIG. 7A) before the reaction and the state (FIG. 7B) after the reaction of two kinds of the nucleic acids for detection used in the method shown in FIG. 5. The states before and after the reaction in the MIP method are shown in FIGS. 8A and 8B, respectively. The mode of hybridization between the nucleic acid for detection and genome DNA in FIG. 8B and amplification procedure of the tag are shown in FIGS. 10A to 10D. The state (FIG. 9A) before the reaction and the state (FIG. 9B) after the reaction in the Golden Gate method are also shown.

As has been illustrated for each example, all the structures of the nucleic acids for detection used in the Barany's zip code method, the method by Sueyama et al., the MIP method and the Golden Gate method by Illumina have common primer regions at two sites, and provide a gene to be detected or a tag sequence in 1:1 correspondence to the mutation of the gene at least at one site. Accordingly, the above-mentioned effect of the invention may be obtained by using a primer group complementary to the tag sequence according to the method of the invention without using one of the common primers.

The amount of the tag primer may be changed depending on the amplification rate of the tag in order to make the amplification rate uniform. Alternatively, the asymmetric PCR may be executed by increasing the amount of either one of the primers.

The tag sequence primer may be modified with a fluorescent pigment, or the common primer may be modified with another fluorescent pigment. Each primer may be modified with a chemical other than the fluorescent pigment such as DIG (Digoxigein), biotin or hapten.

The common primer sides may be classified into plural kinds, preferably about 10 kinds, and may be mixed for the PCR reaction. Since the number of the tags capable of being discriminated is represented by (the number of the kinds of the tags) x (the number of the kinds of the common primers), the number of the kinds of the tags may be remarkably increased.

### Example

The effect of the method of the invention will be described below by suggesting the difference between amplification of the tag with the common primer and amplification of the tag with the tag mix primer.

The tag was used in the multiplex SNP detection method, and the detection reaction was performed according to the method in Japanese Patent Application No. 2004-296784 (Jpn. Pat. Appln. KOKAI Publication No. 2006-101844).

An anonymous human genome sample from the Human Science Research Resources Bank of the Japan Health Science Foundation was used in the example, and 96 SNPs were detected.

Typing by the method of the invention was performed in the following order.
1. Acquisition of sequences and samples
2. Amplification of genome DNA
3. Encode reaction
4. Amplification reaction
5. Detection

The experimental procedure will be described below.

The nucleotide sequences of 96 SNPs in total obtained from the Japanese SNP database JSNP prepared by the Institute of Medical Science, the University of Tokyo, were used as the nucleotide sequences of SNPs to be detected. The detected samples were human genome DNAs extracted from established strains of the human peripheral blood cells distributed by the Human Science Research Resources Bank of the Japan Health Science Foundation. Four samples of PSCDA0503, PSCDA0328, PSCDA0719 and PSCDA0785 were detected.

The method for treating the genome sample will be described in detail below.

A region containing target SNPs was amplified at first by the multiplex PCR from the genome DNAs. Since 24 cites of SNP are amplified in one reaction tube, one sample was subjected to amplification reaction four times in total. The procedure was as follows:

The composition of the solution used for the PCR reaction was as follows. Titanium Taq as a product of Takara Bio was used for the reaction solution. Ultra-pure water was prepared using Milli-Q Synthesis manufactured by Millipore. The primer sequences were designed using Visual OMP manufactured by DNA Software, USA.

| | |
|---|---|
| Titanium Taq (50 fold concentration) | 0.4 µL |
| Titanium Taq Buffer (10 fold concentration) | 2 µL |
| 24 primers (100 µm) | 0.74 µL |
| MgCl₂ (25 mM) | 2.4 µL |
| dNTP (10 mM) | 1.6 µL |
| Genome DNA (5 ng/µL) | 2 µL |
| Ultra-pure water | 13.26 µL |
| Total | 20 µL |

Thermal cycles for the reaction were as follows. PTC-200 manufactured by MJ Research was used as a thermal cycler.

| | | | |
|---|---|---|---|
| 1. | Pre-heating | 94°C | 3 minutes |
| 2. | Denaturation | 94°C | 15 seconds |
| 3. | Elongation | 73°C | 1 minute |

[Steps 2 and 3 were repeated 50 cycles, and the temperature of the elongation step was reduced by 0.1°C and elongation time was prolonged by 5 seconds for each cycle]

### 4. Preservation 10°C fixed temperature

The PCR product of the genome containing the target SNP was obtained by the above-mentioned steps.

While the amplification reaction was divided into four steps, the reaction may be performed at once. When the sample is subjected to one reaction, 94 primers may be amplified in one tube. The 94 primers used in this example correspond to the primers having SEQ ID NOS: 1 to 94.

Then, the primers were subjected to an encode reaction. Since Taq ligase of New England Biolab was used as the ligase, an attached 10 x buffer was used.

| | |
|---|---|
| Probe mix (a mixture for detecting 96 SNPs containing 0.1 µM of each probe) | 0.2 µL |
| 10 x Taq DNA ligase reaction buffer | 3 µL |
| Taq DNA ligase (40 U/µL) | 0.5 µL |
| PCR product (a mixture of four reaction solutions) | 4 µL |
| Ultra-pure water | 22.3 µL |
| Total | 30 µL |

An encode reaction solution was prepared based on the above-mentioned mixed solution. Taq ligase manufactured by New England Biolab was used.

The encode reaction solution was made to react at the following temperatures. A thermal cycler PCT-200 was used for heating.

| | | | |
|---|---|---|---|
| 1. | Denaturation | 95°C | 1 minute |
| 2. | Ligase reaction | 58°C | 60 minutes |
| 3. | Preservation | 10°C | fixed temperature |

The probe ligation reaction by the ligase of the encode reaction is completed by the above-mentioned reaction. Subsequently, a biotinated common probe and a linked query probe and common probe were trapped on streptavidin magnetic beads. The composition of the solution was as follows.

| | |
|---|---|
| Encode product | 30 µL |
| 2 x B&W buffer | 16.5 µL |
| Streptavidin magnetic beads | 5 µL |

The magnetic beads coated with streptavidin on the surface were M-280 magnetic beads manufactured by Dynal, and the solution with the following composition was used as the B&W buffer according to the instruction of the beads.

| | |
|---|---|
| Tris-HCl (pH7.5) | 10 mM |
| EDTA | 1 mM |
| NaCl | 0.2 M |

5 µL of the streptavidin magnetic beads (referred to as magnetic beads hereinafter) was sampled from a shipped original solution containing an antiseptic, and the volume was re-adjusted to 5 µL by replacing the original solution with B&W. The solution thus prepared is shaken at room temperature for 15 minutes so that the magnetic beads are thoroughly dispersed in the solution.

After the shake, the magnetic beads are washed by the following procedure.
1. The magnetic beads are coagulated with a magnet to remove the B&W buffer.
2. The beads are re-dispersed in 100 µL of 1 x B&W at room temperature, followed by pipetting, after which the beads are coagulated with the magnet in order to repeat washing twice for removing the B&W buffer.
3. The beads are re-dispersed in 100 µL of 0.2N aqueous NaOH solution at room temperature, and are shaken with a shaker for 4 minutes.
4. The magnetic beads are coagulated with the magnet to remove the aqueous NaOH solution.
5. The beads are washed twice with 100 µL of 10 mM Tris-HCl at room temperature by the same procedure as in step 2.

Magnetic beads from which non-specifically adhered DNA was removed were obtained by the washing procedure. The magnetic beads are referred to as encoded magnetic beads.

Subsequently, asymmetric PCR was performed in the amplification reaction to amplify and label the tag. SD is a sequence corresponding to primer 1, and Cy3-rED and Cy5-rED' are sequences corresponding to complementary strands of two sequences related to alleles inserted at the position of primer-2, where 5'-terminals of the sequences are labeled with fluorescent pigments. Since Ex Taq manufactured by Takara Bio was used for the polymerase, a buffer of 10 fold concentration and a dNTP mixture attached to the kit were used for preparation. The buffer of 10 fold concentration used contained 20 mM of magnesium ions. The primer was diluted with ultra-pure water.

[For common primer]

| | |
|---|---|
| Primer SD (10 µM) | 0.1 µL |
| Primer Cy3-rED (10 µM) | 0.5 µL |
| Primer Cy5-rED' (10 µM) | 0.5 µL |
| 10 x Ex Taq buffer | 5 µL |
| Ex Taq | 0.5 µL |
| dNTP mixture (10 mM) | 4 µL |
| Ultra-pure water | 38.4 µL |
| Total | 50 µL |

[For tag mix primer]

| | |
|---|---|
| Tag mix primer (25 µM) | 1 µL |
| Primer Cy3-ED (10 µm) | 0.5 µL |
| Primer Cy5-ED' (10 µm) | 0.5 µL |
| 10 x Ex Taq buffer | 5 µL |
| Ex Taq | 0.5 µL |
| dNTP mixture (10 mM) | 4 µL |
| Ultra-pure water | 39.4 µL |
| Magnetic beads | all the amount |
| Total | 50 µL |

The reaction solution was mixed with encoded magnetic beads after removing the solution from the beads, and then the beads were dispersed. Thermal cycle of the reaction was as follows. PTC-200 was used as the thermal cycler. When the cycle elongation method is used, the number of the cycles is preferably 30 to 40 cycles.

| | | | |
|---|---|---|---|
| 1. | Denaturation | 94°C | 1 minute |
| 2. | Denaturation | 94°C | 30 seconds |
| 3. | Annealing | 55°C | 6 minutes |
| 4. | Elongation | 72°C | 30 minutes |
| (40 cycles from step 2 to step 4) | | | |
| 5. | Cooling | 10°C | fixed temperature |

The amplification reaction was completed by the above-mentioned procedure. The product is referred to as a labeled PCR product.

Finally, a detection reaction was performed. A capillary array (Jpn. Pat. Appln. KOKAI Publication No. 11-75812) was used for detection. The capillary array resembles a DNA microarray, and is a device for detecting the nucleic acid by hybridization. Probes are spotted along a flow passageway groove. Probes for detecting the tag are immobilized at 50 points in one groove in the capillary array used in this example, and the volume of the groove was 25 µL. The capillary is formed on a silicon rubber plate, which is bonded to a slide glass, on which the probes are linearly spotted, by taking advantage of adhesivity of the silicon rubber. Holes penetrating to the surface opposed to the groove are open at both ends of the groove, and injection and removal of the sample solution are possible through the penetration holes when the surface of the silicon rubber on which the groove is formed is bonded to the slide glass. A Hubble slide sold by Takara Bio was used for the slide glass for immobilizing the probe, and SP-BIO manufactured by Hitachi Software Engineering was used for spotting of the probe. The silicon rubber was bonded to the slide glass in advance so that the groove matches the spots on the slide glass.

The labeled PCR product was hybridized with the capillary array. Since the magnetic beads remain in the asymmetric PCR solution, the supernatant was collected after allowing the magnetic beads to precipitate so that the magnetic beads are not sampled.

The labeled PCR product (10 µL) per one lane was added to the hybridization solution, and a two-hold dilution hybridization solution was added to the remaining lanes until the concentration reached the final concentration (0.5 x SSC, 0.1% SDS, 15% formamide and 1 mM EDTA). Labeled control target oligonucleotide (2.5 mM, 2 µL) for hybridization was mixed to the 100^{th} probe for normalizing the fluorescence intensity. This solution was heated at 95°C for 1 minute, and 24 µL of the solution was injected to the capillary array placed on a light-shielded hot plate at 37°C for hybridization for 60 minutes.

The capillary array was washed as follows after hybridization.
1. The capillary array is removed from the glass slide in 0.1 x SSC and 0.2% SDS.
2. The glass slide is shaken for 5 minutes in 0.1 x SSC and 0.2% SDS at room temperature.
3. The glass slide is shaken in ultra-pure water for 1 minute at room temperature.
4. The glass slide is dried by air spraying or centrifugation.

The hybridization reaction was completed by the above-mentioned procedure, and florescence was detected with a microarray scanner. GenePix 4000B manufactured by Axon was used for the scanner, and fluorescence was detected at a wavelength for detecting Cy3 and Cy5.

The same amounts of fluorescence-labeled control targets, which hybridize with probes different from the sample, were labeled with Cy3 and Cy5, respectively, were added during hybridization, and were hybridized when the sample was hybridized. The average of the normalized fluorescence intensity is obtained by using the signal intensity as an average value of a sum normalized by dividing the fluorescence intensities of Cy5 and Cy3 of respective SNPs by the fluorescence intensities of respective hybridization control targets. The relation is represented by the formula: avg (SNP_n, sample (Cy5)/HybriCont (Cy5) + SNP_n, sample (Cy3)/HybriCont (Cy3)). [SNP_n, sample (Cy5)] denotes the Cy5 fluorescence intensity of a sample with a SNP No. of n, and HybriCont (Cy5) denotes the detected fluorescence intensity of the Cy5-labeled quantification control target.

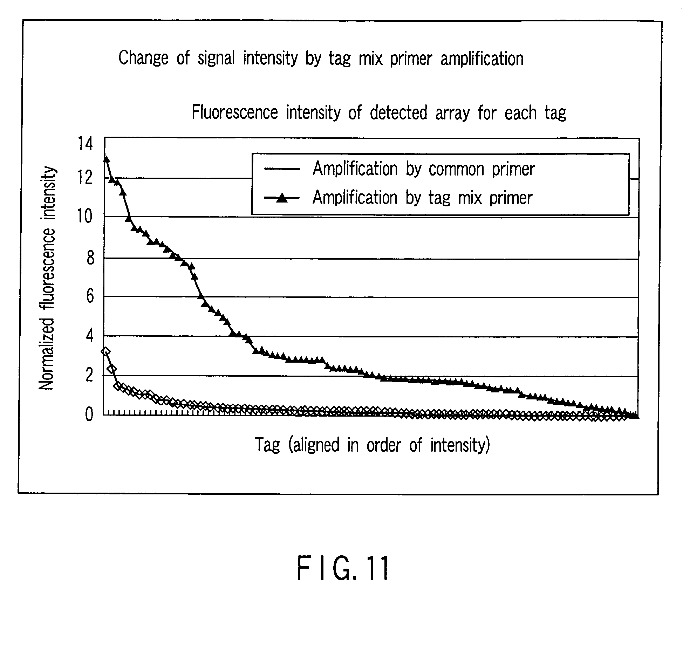

In FIG. 11, the average of the normalized fluorescence intensity was calculated for each SNP, and was plotted in order of higher intensities. The average fluorescence intensity was increased 10 times in the tag mix primers, while the %CV value that represents distribution of the signal intensity was reduced from 160% to 90%, which indicates an improvement in uniformity of amplification.

**Table 1 Amplification method, and fluorescence intensity and distribution of the PCR product obtained**

| | Normalized fluorescence intensity average | %CV value |
|---|---|---|
| Common primer | 0.32 | 160% |
| Tag mix primer | 3.4 | 90% |

The results of detailed analysis of the date shown herein are shown below. Optiplex GX150 manufactured by Dell was used as a computer, and a scattergram was created using Access 2000 as a data base software of Microsoft.

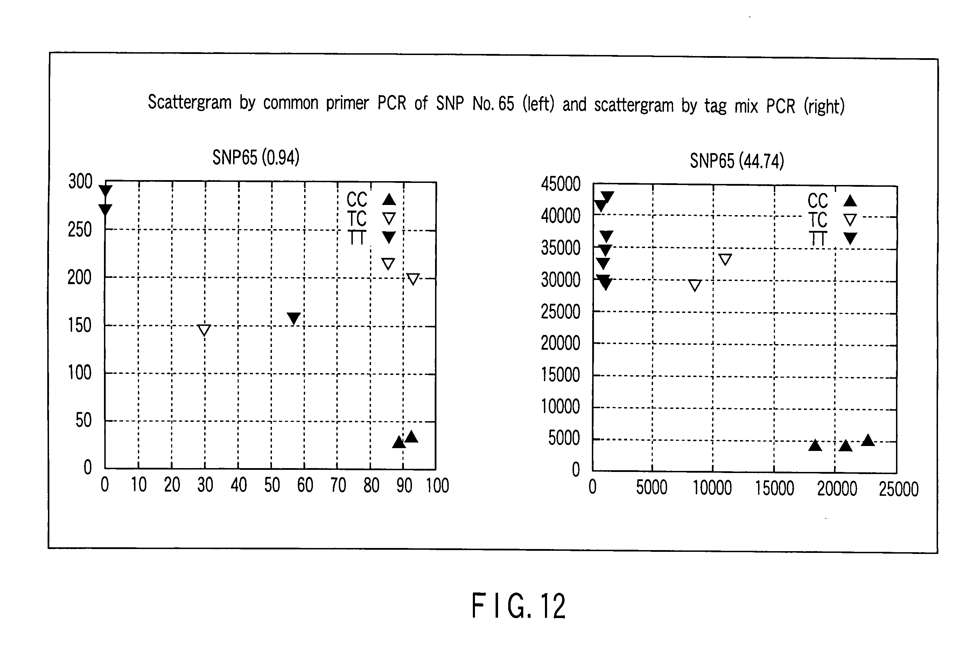

The maximum values of the scales of the X-axis and Y-axis of the scattergram were defined as temporary maximum fluorescence intensities using the signal intensity as an index, and the intensities were listed with respect to 96 SNPs. QV values (the QV value refers to an evaluation value of a cluster of the scattergram defined in Japanese Patent Application No. 2006-249837, and the larger value shows better separation of the cluster) as evaluation of the scattergram were also investigated. As a result, the maximum value of the signal in Table 1 was increased twice or more on average. FIG. 12 shows an example of improvement in the scattergram in SNP No. 65. The signal intensity was remarkably increased by changing the amplification method to enable the cluster to be accurately separated.

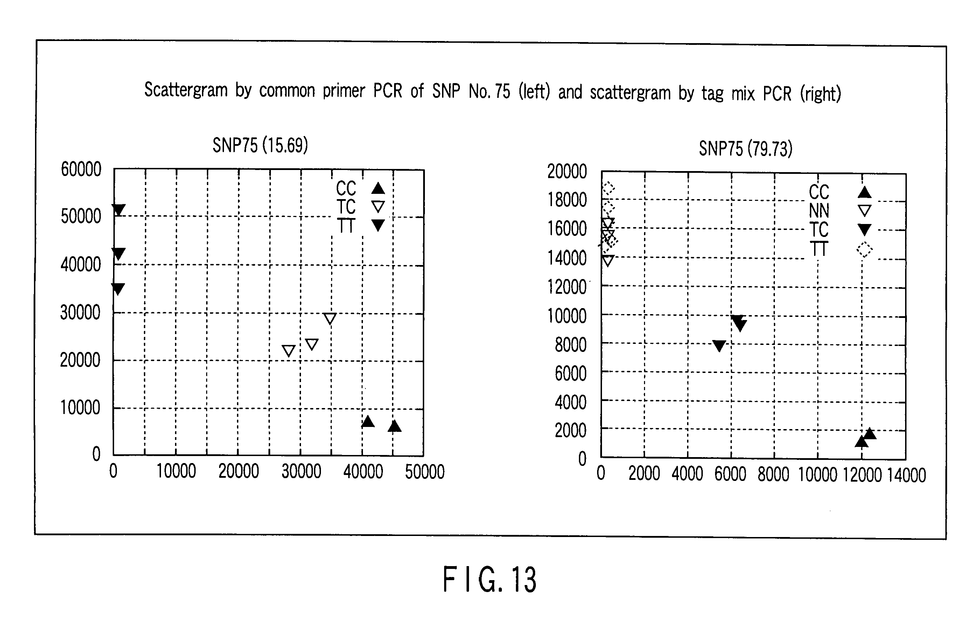

The maximum values of the signals of many SNPs were improved by changing the amplification method. Of 96 SNPs, the X-axis signals were improved in 75 SNPs, and the Y-axis signals were improved in 72 SNPs. However, the signals in both axes were decreased in about 20 SNPs. This seems that, while the common primers were subjected to abnormally strong amplification as in SNP No. 75 in FIG. 13, the amount of the primers available for amplification was restricted in tag mix PCR and the amount of amplification remained in a proper range to exhibit a homogenizing action of the amount of amplification. There was only one SNP that showed a decrease in quality of the scattergram due to reduction of the signal.

The quality of the scattergram is slightly improved by improving the amplification method. The QV value represents the degree of separation of the scattergram. Wile the number of SNPs showing sufficient separation with a QV value of 10 or more was 33 in common primer PCR, the number was increased to 35 in tag mix PCR.

**Table 2**

| | Common primer PCR | | Tag mix PCR | |
|---|---|---|---|---|
| | X-axis | Y-axis | X-axis | Y-axis |
| Average value of maximum values | 5,381 | 7,918 | 12,549 | 20,034 |

In summary, it may be considered that the primers used for PCR amplification may be approximately uniformly supplied to every tag by converting into the tag mix primer, and uniformity of amplification is increased.

The detected intensity still remains dispersed because the steps of PCR for amplifying the genome containing each SNP, detection reaction with ligase and retrieval onto the magnetic beads are necessary before tag mix PCR of the invention, and the amounts of the amplification object were naturally dispersed before PCR due to a difference in the reaction efficiency of each tag (each SNP) in these steps.

The example as hitherto described is only an example for elucidating the effect of the invention, and it is to be understood that the invention is by no means restricted to the example. The references in the specification of the invention are incorporated herein by reference.

As described above, an aspect of the invention provides a method for amplifying plural kinds of nucleic acids for discrimination.

Another aspect of the invention provides a method in which dispersion of the amplification rates among the tags is smaller in the multiplex detection using the tag.

### [Explanation of Reference Numerals]

1 Nucleic acid for detection
2 Nucleic acid for detection
3 Common primer sequence
4 Nucleic acid tag for discrimination
5 Complementary sequence of test gene
6 Common primer sequence
7 Complementary sequence of test gene
8 Nucleotide complementary to SNP
21 Nucleic acid fragment
22 First common primer joint
23 Nucleic acid sequence for discrimination
24 Complementary sequence of first gene
25 Complementary sequence of second gene
26 Second common primer joint
27 First common primer
28 Second common primer
31 Nucleic acid fragment
32 Nucleic acid sequence for discrimination
33 Complementary sequence of first gene
34 Complementary sequence of second gene
35 Nucleic acid sequence for amplification
36 Common primer
37 Mixture of all nucleic acid primers for discrimination
42 Plural primers
43 PCR product
44 Oligonucleotide group
45 Labeled primer
46 SNP
47 Magnetic beads
48 Tag mix primer
49 First labeled common primer
49b Second labeled common primer
50 Linked oligonucleotide
51 Fluorescence-labeled PCR product
53 Microarray
53 Probe immobilization region
54 Immobilized probe
55 Fluorescence-labeled PCR product
60 Common primer
61a First labeled common primer
61b Second labeled common primer
70, 72 Nucleic acid for detection
73 First common primer sequence
74 Nucleic acid tag for discrimination
75 Complementary sequence of test gene
77 Complementary sequence of second test gene
78 Second common primer
100 Cleaved sequence
101 Genome DNA

## Claims

1. A method for amplifying plural nucleic acid fragments,
the nucleic acid fragment comprising one nucleic acid sequence for discrimination selected from a first group consisting of first to n-th nucleic acid sequences for discrimination and one nucleic acid sequence for amplification selected from a second group consisting of first to m-th nucleic acid sequences for amplification,
the method comprising amplifying a mixture of nucleic acid fragments containing plural kinds of the nucleic acid fragments under a condition capable of amplifying the nucleic acids using, as primers, at least a part of the sequences of all the nucleic acid sequences for discrimination that may exist in the mixture of the nucleic acid fragments, and sequences complementary to at least a part of the sequences of the nucleic acid sequences for amplification or sequences at the same strand side as the nucleic acid sequences for amplification,
wherein n is an integer of 2 or more, the first to n-th nucleic acid sequences for discrimination contained in the first group have different nucleotide sequences from one another, m is an integer from 1 or more to n or less, and the first to m-th nucleic acid sequences for amplification contained in the second group have different nucleotide sequences from one another.

2. The method according to claim 1, wherein the amplification is PCR amplification.

3. The method according to any one of claims 1 and 2, wherein the nucleic acid sequence for discrimination is a tag nucleic acid, and the amplification reaction is used as a method for amplifying the tag for multiplex detection.

4. The method according to claim 3, further comprising:
acquiring a sample containing an nucleic acid as an object to be analyzed;
amplifying the nucleic acid as an object to be analyzed;
obtaining a mixture of nucleic acid fragments containing plural kinds of nucleic acid fragments containing one nucleic acid sequence for discrimination selected from a first group consisting of first to n-th nucleic acid sequences for discrimination and one nucleic acid sequence for amplification selected from a second group consisting of first to m-th nucleic acid sequences for amplification by converting an amplification product obtained by the amplification into a tag sequence; and
amplifying the mixture of nucleic acid fragments using primers for the first to n-th nucleic acid sequences for discrimination and primers for the first to m-th nucleic acid sequences for amplification.

5. The method according to any one of claims 1 to 4, wherein the length of the first nucleic acid sequence for discrimination is the same as the length of the first primer for discrimination, the length of each of the second to n-th nucleic acid sequences for discrimination is also the same as the length of each of the second to n-th primers for discrimination, the length of the first nucleic acid sequence for amplification is the same as the length of the first primer for amplification, and the length of each of the second to m-th nucleic acid sequences for amplification is also the same as the length of each of the second to m-th primers for amplification.

6. The method according to any one of claims 1 to 4, wherein the length of the first nucleic acid sequence for discrimination is 1 to 10 nucleotides longer than the length of the first primer for discrimination, the length of each of second to n-th nucleic acid sequences for discrimination is also 1 to 10 nucleotides longer than the length of each of the second to n-th primers for discrimination, the length of the first nucleic acid sequence for amplification is 1 to 10 nucleotides longer than the length of the first primer for amplification, and the length of each of second to m-th nucleic acid sequences for amplification is also 1 to 10 nucleotides longer than the length of each of the second to m-th primers for amplification.

7. The method according to any one of claims 1 to 6, wherein plural primers from the first to n-th and from the first to m-th primers are contained in different amounts depending on the amounts of complementary nucleic acid sequences for discrimination or the amounts of complementary nucleic acid sequences for amplification.

8. The method according to any one of claims 1 to 7, wherein the primer used in the method is modified by a labeling substance at a position except a 3'-terminal.

9. A reagent kit used for the method according to any one of claims 1 to 8, containing plural primers from the first to n-th primers, common primers, an enzyme for amplifying the nucleic acids, a buffer component and a dNTP mixture.
